# EUROPEAN PATENT APPLICATION

(11) **EP 1 925 619 A1**
(43) Date of publication of application: **28.05.2008**
(21) Application number: 06124539.5
(22) Date of filing: 22.11.2006
(51) Int. Cl.: C07D 491/04

(54) **Preparation of 4-substituted 2-amino-benzo[4,5]furo[3,2-d]pyrimidine derivatives**

(71) Applicant: Cellzome (UK) Ltd., CB10 1XL Cambridgeshire Little Chesterford (GB)
(72) Inventor: Jones, Alison, Cottenham, CB4 8SA (GB); Wilson, Francis, Herts, AL7 AQJ (GB); Dyke, Hazel, Harlow, CM19 5TR (GB); Price, Steven, Harlow, CM19 5TR (GB); Cramp, Sue, Harlow, CM19 5TR (GB); Szabó, András, 1184 Budapest (HU); Repasi, Jozsef, 2030 Erd (HU)
(74) Representative: Huhn, Michael

(57) **Abstract**

The present invention is directed to a process for the preparation of a compound of formula (I) 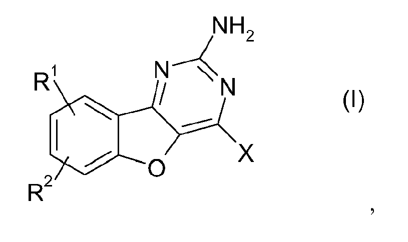 wherein R¹, R² and X have the meaning as indicated in the description and claims. Said compounds are useful as key intermediates for the preparation of the related pharmaceutically active compounds or as active compounds.

## Description

4-Substituted 2-amino-benzo[4,5]furo[3,2-d]pyrimidine derivatives are potential active compounds useful as medicament or prodrug.

For example selected compounds of this class are histamine H4 receptor modulators and therefore useful for the preparation of a medicament for the treatment or prevention of conditions mediated by said receptor. This includes the treatment of inflammatory disorders.

The European patent application with the filing number 06101510.3 discloses such compounds, where in 4-position of the pyrimidine tricycle a heterocycle is attached via a nitrogen ring atom.

Furthermore selected compounds of this class are protein kinase inhibitors and therefore useful for the preparation of a medicament for the treatment or prevention of conditions mediated by said kinases. This includes the treatment of cancer.

WO-A 2005/037825 discloses different classes of compounds including 4-substituted 2-amino-benzo[4,5]furo[3,2-d]pyrimidine derivatives. However, no synthesis is described in detail for this group of compounds.

A key step in the synthesis of said compounds is the formation of the tricycle having a pyrimidine ring with an amino group in 2-position, which is already functionalized at the 4-position as an anchor for the addition of further substituents, like nitrogen containing heterocycles.

In principle, this tricycle can be formed by the intramolecular cyclization of the corresponding 4-substituted 2-amino-5-bromo-6-(2-hydroxyphenyl)-pyrimidine derivative.

However there is a need for alternative routes for the formation of said tricycles, which are more convenient with regard to overall yield, purity and the like.

Thus, an object of the present invention is to provide a process for the preparation of 4-substituted 2-amino-benzo[4,5]furo[3,2-d]pyrimidine derivatives to form compounds suitable as key intermediates for the preparation of the aforementioned active compounds or to form these active compounds.

Accordingly, the present invention provides a process for the preparation of a compound of formula (I) 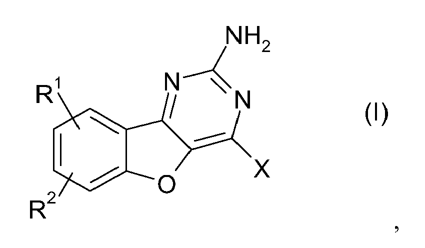 wherein
R¹, R² are independently selected from the group consisting of H; F; Cl; Br; C₁₋₄ alkyl; and OC₁₋₄alkyl, wherein each C₁₋₄ alkyl is optionally substituted with one or more halogen; and
X is OH;
comprising the step of
(a₀) reacting a compound of formula (II) 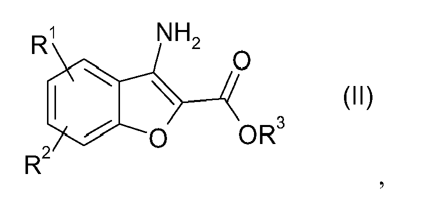 wherein
R¹, R² are independently selected from the group consisting of H; F; Cl; Br; C₁₋₄ alkyl; and OC₁₋₄ alkyl, wherein each C₁₋₄ alkyl is optionally substituted with one or more halogen; and
R³ is C₁₋₄ alkyl;
with chloroformamidine or a salt thereof.

It was found, that the reaction of compounds of formula (II) with chloroformamidine (H₂NC(NH)Cl) or a salt thereof gives the desired 4-substituted tricycle in good yield and purity, wherein the substituent in 4-position is a hydroxyl group which is suitable for further derivatization.

Compounds of formula (I) may also appear at least in part in the keto form.

The groups R¹, R² are independently selected from the group consisting of H; F; Cl; Br; C₁₋₄ alkyl; and OC₁₋₄alkyl.

Within the meaning of the present invention C₁₋₄ alkyl is methyl, ethyl, n-propyl, i-propyl, n-butyl, i-butyl, sec-butyl or tert-butyl. Preferably C₁₋₄ alkyl is methyl or ethyl.

For R¹, R² the C₁₋₄ alkyl or OC₁₋₄ alkyl group is optionally substituted with one or more halogen. In case more halogen atoms are present these can be the same or different.

Within the meaning of the present invention halogen means fluoro, chloro, bromo or iodo, preferably halogen is fluoro or chloro, especially fluoro.

Examples for halogenated groups for R¹, R² are CH₂F, CH₂Cl, CHF₂, CF₃, CF₂CF₃, OCF₃.

In step (a₀) of the process according to the present invention a compound of formula (II) is reacted with chloroformamidine or a salt thereof. Suitable salts are known in the art and include hydrohalides, like hydrochlorides. A convenient method for the preparation of the chloroformamidine includes the reaction of cyanamide with a solution containing hydrogen chloride or with gaseous hydrogen chloride.

In a preferred embodiment the chloroformamidine or a salt thereof is added in step (a ₀) to a solution of the compound of formula (II) in a solvent.

Furthermore it is preferred that the cholorformamidine or a salt thereof is added at a temperature in the range of 50°C to 150°C. It is possible that the temperature varies during the addition. However it is preferred that the temperature is constant. Preferably the temperature is in the range of 80°C to 120°C and even more preferred the temperature is about 100°C ± 10°C, preferably ± 5°C.

After completion of the addition the temperature for the remaining reaction time is preferably in the range of 125°C to 185°C. It is possible that the temperature varies during the remaining reaction time. However it is preferred that the temperature is constant after having reached the desired temperature. Preferably the temperature is in the range of 140°C to 180°C and even more preferred the temperature is about 160°C ± 10°C, preferably ± 5°C.

The temperature during the addition of the chloroformamidine or salt thereof and the temperature for the remaining reaction time may be the same of different. It is preferred that the temperatures are different. Preferably, the difference is at least 10°C, more preferred at least 25°C and even more preferred at least 50°C, wherein the temperature during the addition is the lower one.

The solvent which can be used to solve the compound of formula (II) can be selected to have a boiling point which is high enough to carry out the reaction for the desired temperature.

Suitable solvents are glycols and polyols which optionally include ether bridges and ethers thereof and other high boiling solvents. Solvents with only one component can be used as well as mixtures of different solvents. Examples of suitable solvents are benzonitrile, ethylene glycol, diethylene glycol, triethylene glycol, triethylene glycol dimethyl ether, dimethylsulfoxide, sulfolane or a mixture of at least two of them. It is also possible to use a mixture of three, four or more components of the solvent. A preferred solvent is sulfolane.

The reaction time may vary and is dependent upon the amount of added chloroformamidine or salt thereof. Normally, a reaction time in the range of 5 minutes to 24 hours is sufficient.

Preferably the reaction time is less than 12 hours, more preferred less than 6 hours, even more preferred less than 3 hours.

The compound of formula (II) may be prepared by the cyclization of the respective alkoxycarbonylmethoxy benzonitrile.

Accordingly, in a preferred embodiment the compound of formula (II) is prepared in a preceding step of
(a ₋₁) cyclizing of a compound of formula (III) 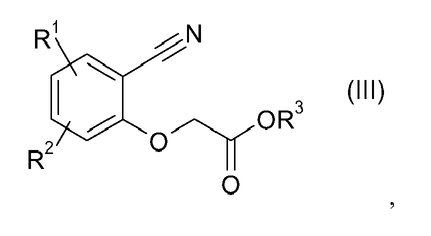 wherein
   R¹, R² are independently selected from the group consisting of H; F; Cl; Br; C₁₋₄ alkyl; and OC₁₋₄ alkyl, wherein each C₁₋₄ alkyl is optionally substituted with one or more halogen; and
   R³ is C₁₋₄ alkyl.

Thus, a preferred process according to the present invention comprises the steps (a ₋₁) and (a ₀).

The cyclization can be carried out by methods generally known in the art. Normally a strong base is used, like alkoxylate salts, e.g. KO^{t}Bu.

The compound of formula (III) may be prepared by the dehydration of the respective acid amide.

Accordingly, in a preferred embodiment the compound of formula (III) is prepared in a preceding step of
(a ₋₂) dehydrating of a compound of formula (IV) 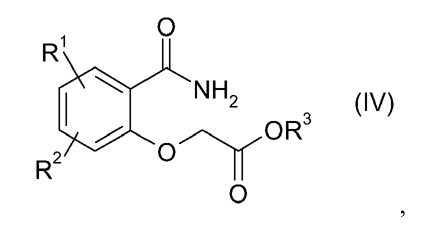 wherein
   R¹, R² are independently selected from the group consisting of H; F; Cl; Br; C₁₋₄ alkyl; and OC₁₋₄alkyl, wherein each C₁₋₄ alkyl is optionally substituted with one or more halogen; and
   R³ is C₁₋₄ alkyl.

Thus, a preferred process according to the present invention comprises the steps (a ₋₂), (a ₋₁) and (a o).

As a dehydration agent it is preferred to use POCl₃. However other suitable dehydration agents are applicable and known in the art for the general conversion of an acid amide to a nitrile. Examples are described in March, Jerry; Advanced Organic Chemistry; Reactions, Mechanisms, and Structure; Wiley 1992; chapter 17, reaction 7-39.

Compounds of formula (IV) are available by reacting the appropriately 2-hydroxybenzamide with alkyl chloroacetate.

It was found that the introduction of the alkoxycarbonylmethoxy group is more convenient when reacting the benzamide with the choroacetate rather than using the corresponding nitrile.

The 2-hydroxybenzamides may be commercially available or readily prepared starting with the corresponding salicylic acids or esters thereof. The preparation of methyl 5-chlorosalicylate is, e.g., described by R.D. Youssefyeh et al., J. Med. Chem. 35 (1992), 895-903.

As mentioned above the key intermediate of formula (I) can be prepared in step (a ₀) of the process according to the present application. These compounds have a hydroxyl group in 4-position to introduce other substituents by way of substitution.

In a preferred embodiment the hydroxyl group in 4-position of the compound of formula (I) (X = OH) is converted into the chloro derivative using POCl₃. This is advantageous for the introduction of nitrogen containing heterocycles via a nitrogen ring atom.

Accordingly, in a preferred embodiment the process according to the present invention further comprises the step of
(a ₁) reacting a compound of formula (I) 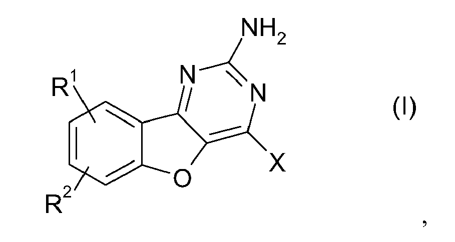 wherein
   R¹, R² are independently selected from the group consisting of H; F; Cl; Br; C₁₋₄ alkyl; and OC₁₋₄alkyl, wherein each C₁₋₄ alkyl is optionally substituted with one or more halogen; and
   X is OH;
   with POC1₃ to give a compound of formula (I) 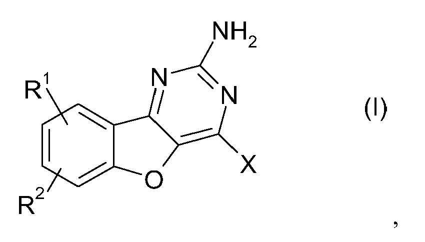 wherein
   R¹, R² are independently selected from the group consisting of H; F; Cl; Br; C₁₋₄ alkyl; and OC₁₋₄ alkyl, wherein each C₁₋₄ alkyl is optionally substituted with one or more halogen; and
   XisCl.

Thus, a preferred process according to the present invention comprises the steps (a ₀) and (a ₁). Yet another preferred process according to the present invention comprises the steps of (a ₋₁), (a ₀) and (a ₁). Yet another preferred process according to the present invention comprises the steps of (a ₋₂), (a ₋₁), (a₀) and (a ₁).

The substitution of the hydroxyl group by a chloro group for comparable heterocyclic aromatic systems (activated systems) is known in the art and described in, e.g., March, Jerry; Advanced Organic Chemistry; Reactions, Mechanisms, and Structure; Wiley 1992; chapter 13, reaction 3-8.

Compounds of formula (I), wherein X is chloro are suitable intermediates to introduce nitrogen containing heterocycles by way of substitution. This may lead to active compounds, probably after activation/deactivation and/or protection/deprotection of functional groups which are suitable for the preparation of a medicament.

Accordingly, in a preferred embodiment the process according to the present invention further comprises the steps of
(a₂) reacting a compound of formula (I) 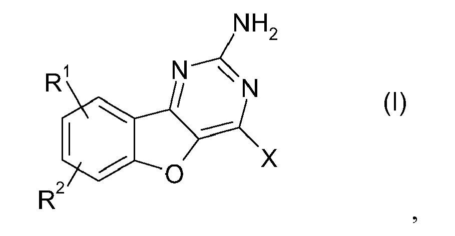 wherein
   R¹, R² are independently selected from the group consisting of H; F; Cl; Br; C₁₋₄ alkyl; and OC₁₋₄ alkyl, wherein each C₁₋₄ alkyl is optionally substituted with one or more halogen; and
   X is Cl;
   with a compound of the formula (V) 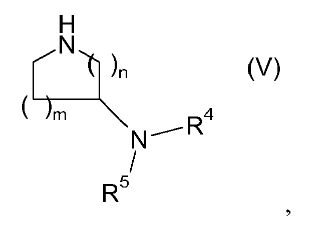 wherein
   R⁴, R⁵ are independently selected from the group consisting of H; C₁₋₄ alkyl; and C₃₋₆ cycloalkyl, wherein each C₁₋₄ alkyl, C₃₋₆ cycloalkyl is optionally substituted with one or more halogen;
   Optionally at least one of R⁴, R⁵ is a protective group resistant to the reaction conditions of step (a₂);
   n is 1 or 2;
   m is 0, 1 or 2; and
(a ₃) optionally removing the protective group;
to give a compound of formula (VI) 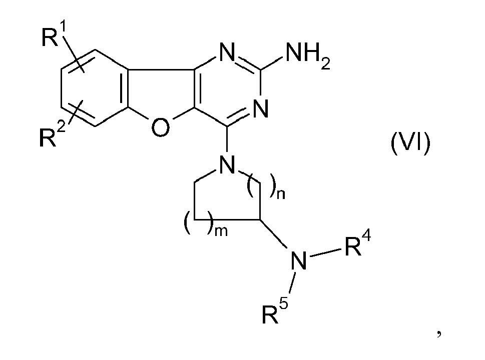 wherein
R¹, R² are independently selected from the group consisting of H; F; Cl; Br; C₁₋₄ alkyl; and OC₁₋₄alkyl, wherein each C₁₋₄ alkyl is optionally substituted with one or more halogen; and
R⁴, R⁵ are independently selected from the group consisting of H; C₁₋₄ alkyl; and C₃₋₆ cycloalkyl, wherein each C₁₋₄ alkyl, C₃₋₆ cycloalkyl is optionally substituted with one or more halogen;
n is 1 or 2; and
m is 0, 1 or 2.

Thus, a preferred process according to the present invention comprises the steps (a ₀), (a ₁), (a ₂) and optionally (a ₃). Yet another preferred process according to the present invention comprises the steps of (a -₁), (a ₀), (a ₁), (a ₂) and optionally (a ₃). Yet another preferred process according to the present invention comprises the steps of (a ₋₂), (a ₋₁), (a ₀), (a₁), (a ₂) and optionally (a ₃).

The groups R⁴, R⁵ are independently selected from the group consisting of H; C₁₋₄ alkyl; and C₃₋₆ cycloalkyl.

Within the meaning of the present invention C₃₋₆ cycloalkyl is cyclopropyl, cyclobutyl, cyclopentyl, cyclohexyl or cycloheptyl. Preferably C₃₋₆ cycloalkyl is cyclopropyl.

For R⁴, R⁵ the C₁₋₄ alkyl or C₃₋₆ cycloalkyl group is optionally substituted with one or more halogen. In case more halogen atoms are present these can be the same or different.

Within the meaning of the present invention halogen means fluoro, chloro, bromo or iodo, preferably halogen is fluoro or chloro, especially fluoro.

Examples for halogenated groups for R⁴, R⁵ are CH₂F, CH₂CI, CHF₂, CF₃, CF₂CF₃, or 2-fluoro-cycloprop-1-yl.

Optionally at least one of R⁴, R⁵ is a protective group resistant to the reaction conditions of step (a₂). Suitable protective groups for an amino group are known in the art and described in, e.g., Greene, Theodora W; Wuts, Peter G; Protective Groups in Organic Synthesis; Wiley 1999. An example for a suitable protective group is the tert.-butyloxycarbonyl (BOC) group.

In case a protective group is used step (a₂) is followed by an additional step (a₃) to remove the protective group, if desired.

Compounds of formula (VI) are suitable as histamine H4 receptor modulators.

### Examples

### Example 1 Synthesis of methyl 5-chlorosalicylate

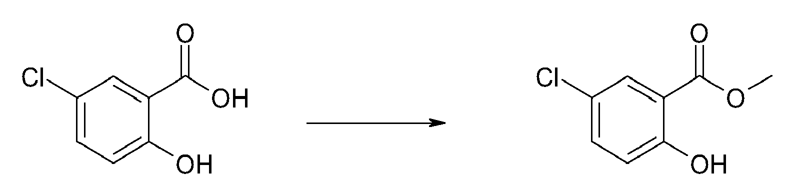

To 750 ml methanol (MeOH) is added thionyl chloride (165.0 ml, 269.11 g, 2.26 mol, 1.3 eq) dropwise maintaining the temperature within the range from (+15)-(+20) °C. After the addition is complete, the solution is treated with 5-chlorosalicylic acid (300 g, 1.74 mol, 1.0 eq) and heated at reflux overnight and a small amount of thionyl chloride (16.5 ml, 26.91 g, 226.13 mmol, 0.13 eq) is added to reach full conversion and stirred further at reflux temperature for an hour. The cooled reaction mixture is concentrated under vacuum to give a beige solid.

This material is used in the next step without purification.

### Example 2 Synthesis of 5-chloro-2-hydroxybenzamide

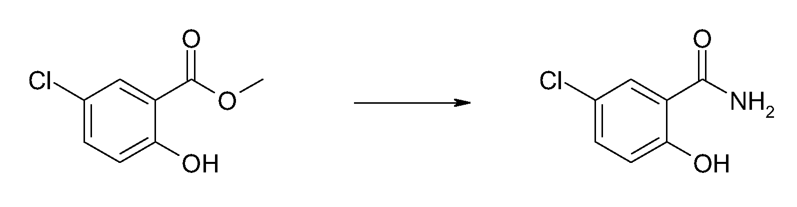

To a solution of ammonia (400 ml, 25%) is added methyl 5-chlorosalicylate (87.7 g, 470.0 mmol, 1 eq) in few portions between (0)-(+10) °C. Thus obtained white suspension is stirred further at room temperature overnight, a clear solution is obtained which is cooled down and acidified to pH=6 with acetic acid (300 ml). The precipitated material is filtered off, washed with water and dried in vacuo at 60 °C to almost dryness then suspended in 2-propanol (200 ml), filtered and dried (vacuo, 60°C), a beige solid is obtained (58.6 g, 341.53 mmol, 72.67%).

¹H NMR (DMSO-d₆): 6.92 (d, 1H), 7.43 (dd, 1H), 7.95 (d, 1H), 8.05 (br s, 1H), 8.47 (br s, 1H); DMSO = dimethylsulfoxide

### Example 3 Synthesis of 5-chloro-2-(methoxycarbonylmethoxy)benzamide

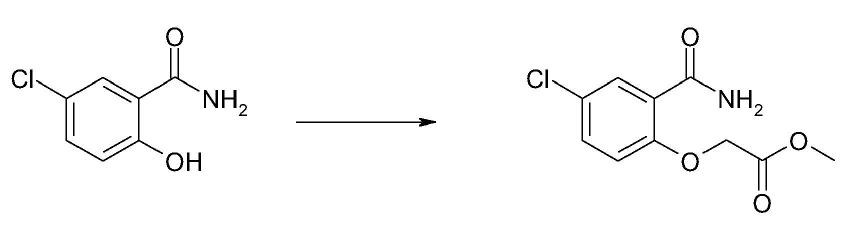

5-Chloro-2-hydroxybenzamide (87.31 g, 508.86 mmol, 1 eq) is dissolved in 330 ml of dry N,N-dimethyl formamide (DMF) K₂CO₃ (105.5 g, 763.33 mmol, 1.5 eq) then methyl chloroacetate (48.98 ml, 60.64 g, 558.79 mmol, 1.1 eq) is added at room temperature. The reaction mixture is stirred overnight; then it is poured onto ice-water (1200 ml) and the precipitate is filtered, washed with 2-propanol and dried in vacuo at 60 °C. A white solid is obtained (80.61 g, 330.84 mmol, 65.02%).

¹H NMR (DMSO-d₆): 3.73 (s, 3H), 4.98 (s, 2H), 7.15 (d, 1H), 7.52 (dd, 1H), 7.82 (m, 2H), 7.99 (brs, 1H)

### Example 4 Synthesis of 5-chloro-2-(methoxycarbonylmethoxy)benzonitrile

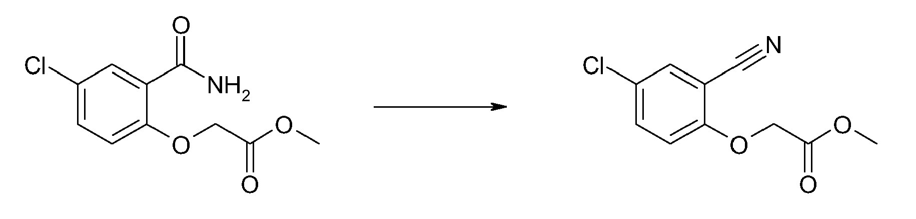

To a suspension of 5-chloro-2-(methoxycarbonylmethoxy)benzamide (80.61 g, 330.84 mmol, 1 eq) in acetonitrile (250 ml) is added POC1₃ (47.8 ml, 78.63 g, 496.62 mmol, 1.5 eq) at reflux temperature and the reaction mixture is heated at reflux for half an hour. The cooled reaction mixture is poured into ice-water (300 ml), the precipitated white product is filtered, washed with water (2x100 ml) and dried in vacuo at 65 °C. The nitrile (63.2 g, 280.10 mmol, 84.66 %) is obtained as a white crystalline compound.

¹H NMR (DMSO-d₆): 3.71 (s, 3H), 5.06 (s, 2H), 7.23 (d, 1H), 7.69 (dd, 1H), 7.93 (d, 1H)

### Example 5 Synthesis of 3-amino-5-chlorobenzofuran-2-carboxylic acid methyl ester

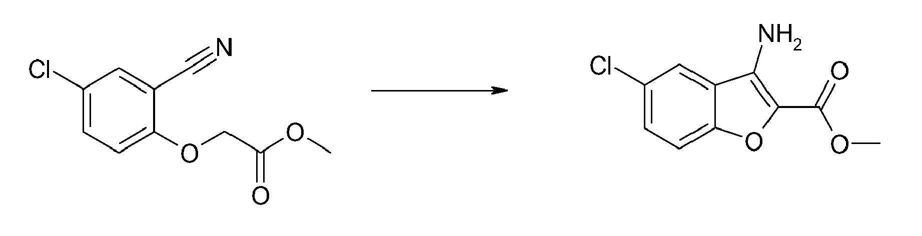

To a suspension of potassium tert.-butanolate (KO^{t}Bu) (5.22 g, 46.52 mmol, 1.05 eq) in 200 ml dry tetrahydrofuran (THF) is added carefully a solution of 5-chloro-2-(methoxycarbonylmethoxy)benzonitrile (10.00 g, 44.32 mmol, 1.0 eq) in dry THF (200 ml) over a period of half an hour maintining the temperature at 0-10 °C. After the addition is complete, the progress of the reaction is monitored by TLC until it is complete. The reaction mixture is poured onto ice (400 g), acidified with 2M HCl solution to pH=7 and diluted with water (1600 ml), the product is precipitated and filtered off. A light purple solid (6.95 g, 30.80 mmol, 69.49%) is obtained, mp.: 152.7-153 °C.

¹H NMR (CDCl₃): 3.97 (s, 3H), 4.98 (br s, 2H), 7.38 (m, 2H), 7.54 (m, 1H); CDCl₃ = chloroform-d₁

### Example 6 Synthesis of chloroformamidine hydrochloride

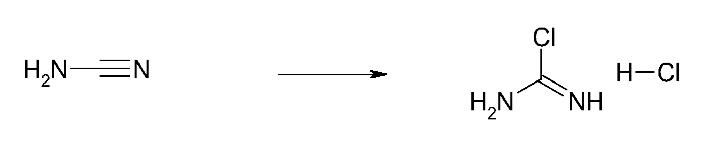

A solution of cyanamide (50.0g, 1.19 mol, 1 eq) in diethylether (900 ml) is cooled to 0 °C and then hydrogen chloride in diethyl ether (450 g, 29%) is added maintaining the temperature below +5 °C. After the addition is complete, the obtained white suspension is stirred further at room temperature for a short time, the white precipitate is filtered off and dried in vacuo at 30 °C. A white solid (132.66 g, 1.15 mol, 96.64%) is obtained.

### Example 7 Synthesis of 2-amino-8-chloro-3H-benzo[4,5]furo[3,2-d]pyrimidin-4-one

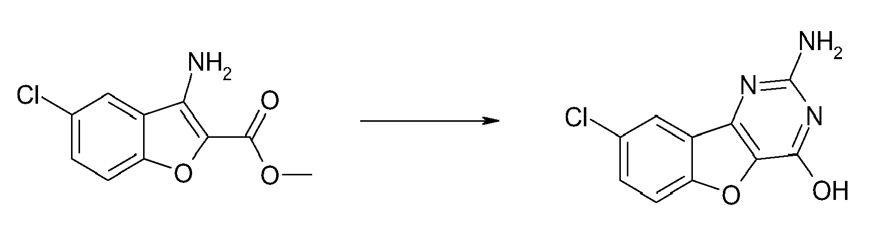

To a solution of 3-amino-5-chlorobenzofuran-2-carboxylic acid methyl ester (5.0 g, 22.16 mmol, 1 eq) in sulfolane (25 ml) is added chloroformamidine hydrochloride (3.82 g, 33.24 mmol, 4.5 eq) at 100 °C and the reaction mixture is stirred further at 160 °C for an hour, it is cooled down to 50 °C and diluted with methanol (75 ml), the precipitated product is filtered off, dried in vacuo at 80°C. A yellowish solid (3.85 g, 16.34 mmol, 73.73%) is obtained.

¹H NMR (DMSO-d₆): 7.67 (dd, 1H), 7.82 (d, 1H), 8.06 (d, 1H)

### Example 8 Synthesis of 2-amino-4,8-dichlorobenzo[4,5]furo[3,2-d]pyrimidine

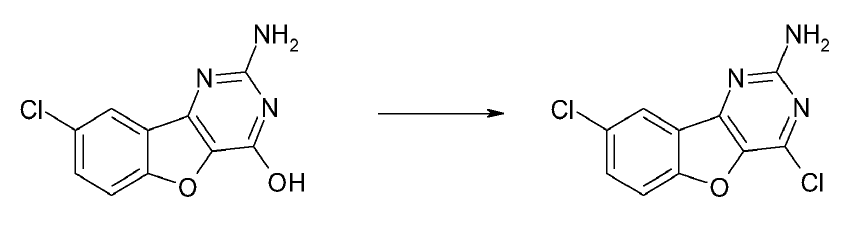

2-Amino-8-chloro-3H-benzo[4,5]furo[3,2-*d*]pyrimidin-4-one (5.0 g, 21.2 mmol, 1.0 eq) and tetramethylammonium chloride (3.6 g, 31.8 mmol, 1.5 eq) are suspended in acetonitrile (90 ml), cooled to 15°C and phosphorus oxychloride (11.8 ml, 19.3 g, 125.8 mmol, 5.9 eq) is added at 15-20°C (10 min). The resulting mixture is refluxed for 3 hrs. After disappearance of the starting material (montoring by TLC) the reaction mixture is poured onto a mixture of crushed ice and 45 saturated aq. Na₂CO₃ solution (45 ml) and the pH was adjusted to 6-7. The precipitated product is filtered, washed with water, suspended in 2-propanol (20 ml), stirred for 10 min then filtered, the solid material is washed with 2-propanol (10 ml) and dried in *vacuo* at 30°C. A yellowish solid (4.7 g, 87.7%) is obtained.

### Example 9 Synthesis of tert-Butyl [(R)-1-(2-amino-8-chlorobenzo[4,5]furo[3,2-d]pyrimidin-4-yl)pyrrolidin-3-yl] N-methyl carbamate

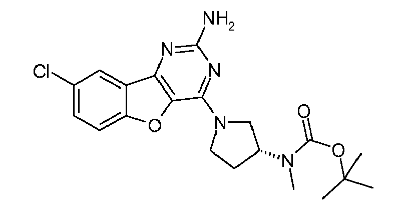

A suspension of2-amino-4,8-dichlorobenzo[4,5]furo[3,2-d]pyrimidine (34.79 g, 136.92 mmol, 1.0 eq) in IPA (550 ml) is treated with N,N-diisopropylethylamine (47.70 ml, 35.39 g, 273.83 mmol, 2.0 eq) followed by a solution of (R)-3-[(N-tert-butoxycarbonyl)-N-methyl] aminopyrrolidine (31.53 g, 137.90 mmol, 1.0 eq, 87.6%) and stirred at room temperature overnight. The reaction mixture is evaporated to dryness, the residue is dissolved in DCM (300 ml) and washed with water (200 ml), saturated NaHCO₃ solution (200 ml) and water (200 ml), the organic layer is dried over Na₂SO4 and evaporated to give a reddish solid (53.60 g, 128.26 mmol, 93. 7%).

¹H NMR (CDCl₃): d 1.5 (s, 9H), 2.1 (m, 1H), 2.2 (m, 1H), 2.85 (s, 3H), 3.75 (br, 2H), 4.1 (br, 2H), 4.8-5.0 (br, 3H), 7.45 (d, 1H), 7.5 (dd, 1H), 8.0 (d, 1H).

### Example 10 [(R)-1-(2-Amino-8-chlorobenzo[4,5]furo[3,2-d]-pyrimidin-4-yl)pyrrolidin-3-yl] N-methyl amine

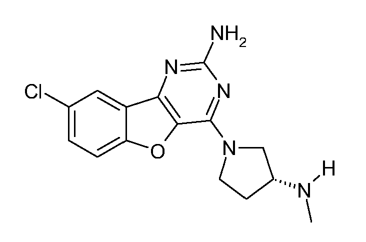

A solution of *tert*-butyl [(R)-1-(2-amino-8-chlorobenzo[4,5]furo[3,2-d]pyrimidin-4-yl)pyrrolidin-3-yl] N-methyl carbamate (2.59g) and trifluoroacetic acid (40mL) in dichloromethane (70mL) is stirred at room temperature overnight. The mixture is evaporated to dryness and the residue is dissolved in methanol and loaded onto an Isolute® SCX-2 column eluting with methanol to remove the unwanted by-products then with a solution of ammonia in methanol (2M) to give the crude product. After evaporation, the residue is recrystallised from IMS to give [(R)-1-(2-amino-8-chlorobenzo[4,5]furo[3,2-d]-pyrimidin-4-yl)pyrrolidin-3-yl] N-methyl amine as an off-white solid.

¹H NMR (DMSO-d₆ 80°C): d 1.8 (m, 1H), 2.1 (m, 1H), 2.35 (s, 3H), 3.3 (m, 1H), 3.6 (m, 1H), 3.8 (m, 1H), 3.9 (m, 2H), 5.7 (br s, 2H), 7.55 (dd, 1H), 7.65 (d, 1H), 7.85 (d, 1H).
LCMS (method A): retention time 3.92 minutes (M+H⁺) 318.

## Claims

1. A process for the preparation of a compound of formula (I) 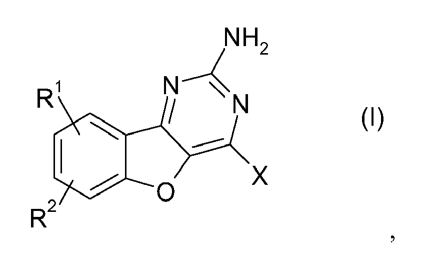 wherein
R¹, R² are independently selected from the group consisting of H; F; Cl; Br; C₁₋₄ alkyl; and OC₁₋₄ alkyl, wherein each C₁₋₄ alkyl is optionally substituted with one or more halogen; and
X is OH;
comprising the step of
(a₀) reacting a compound of formula (II) 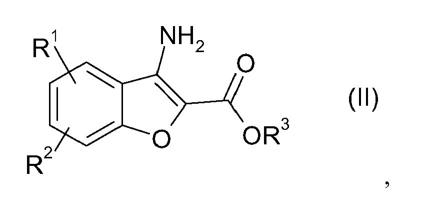 wherein
R¹, R² are independently selected from the group consisting of H; F; Cl; Br; C₁₋₄ alkyl; and OC₁₋₄alkyl, wherein each C₁₋₄ alkyl is optionally substituted with one or more halogen; and
R³ is C₁₋₄ alkyl;
with chloroformamidine or a salt thereof.

2. The process according to claim 1, wherein the chloroformamidine or a salt thereof is added in step (a₀) to a solution of the compound of formula (II) in a solvent.

3. The process according to claim 2, wherein the chloroformamidine or a salt thereof is added at a temperature in the range of 50°C to 150°C.

4. The process according to claim 2 or 3, wherein the reacting after having completely added the chloroformamidine or a salt thereof is carried out at temperature in the range of 125°C to 185°C.

5. The process according any one of claims 2 to 4, wherein the solvent is benzonitrile, ethylene glycol, diethylene glycol, triethylene glycol, triethylene glycol dimethyl ether, dimethylsulfoxide, sulfolane or a mixture of at least two of them.

6. The process according to any one of claims 1 to 5, wherein the compound of formula (II) is prepared in a preceding step of
(a ₋₁) cyclizing of a compound of formula (III) 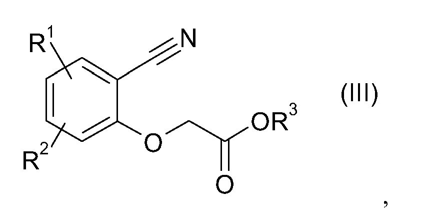 wherein
R¹, R² are independently selected from the group consisting of H; F; Cl; Br; C₁₋₄ alkyl; and OC₁₋₄alkyl, wherein each C₁₋₄ alkyl is optionally substituted with one or more halogen; and
R³ is C₁₋₄ alkyl.

7. The process according to claim 6, wherein the compound of formula (III) is prepared in a preceding step of
(a ₋₂) dehydrating of a compound of formula (IV) 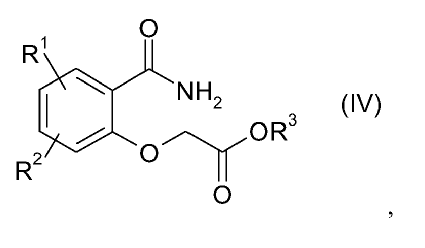 wherein
R¹, R² are independently selected from the group consisting of H; F; Cl; Br; C₁₋₄ alkyl; and OC₁₋₄alkyl, wherein each C₁₋₄ alkyl is optionally substituted with one or more halogen; and
R³ is C₁₋₄ alkyl.

8. The process according to claim 7, wherein the dehydration is carried out using POC1₃.

9. The process according to any one of claims 1 to 8, further comprising the step of
(a₁) reacting a compound of formula (I) 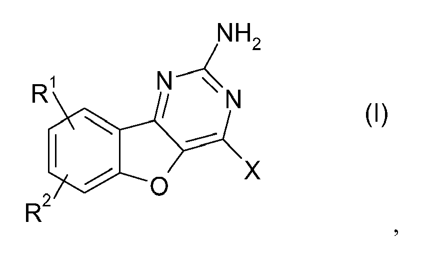 wherein
R¹, R² are independently selected from the group consisting of H; F; Cl; Br; C₁₋₄ alkyl; and OC₁₋₄ alkyl, wherein each C₁₋₄ alkyl is optionally substituted with one or more halogen; and
X is OH;
with POCl₃ to give a compound of formula (I) 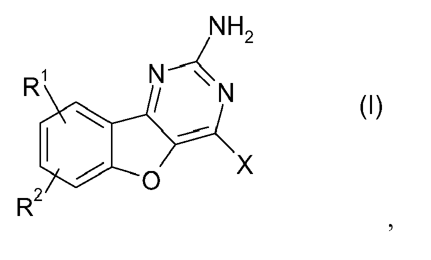 wherein
R¹, R² are independently selected from the group consisting of H; F; Cl; Br; C₁₋₄ alkyl; and OC₁₋₄ alkyl, wherein each C₁₋₄ alkyl is optionally substituted with one or more halogen; and
X is Cl.

10. The process according to claim 9 further comprising the steps of
(a ₂) reacting a compound of formula (I) 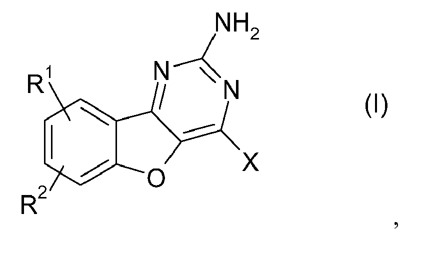 wherein
R¹, R² are independently selected from the group consisting of H; F; Cl; Br; C₁₋₄ alkyl; and OC₁₋₄alkyl, wherein each C₁₋₄ alkyl is optionally substituted with one or more halogen; and
X is Cl;
with a compound of the formula (V) 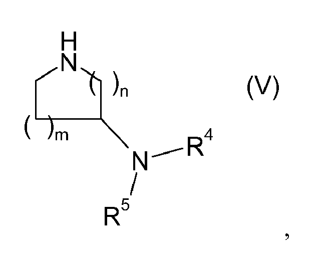 wherein
R⁴, R⁵ are independently selected from the group consisting of H; C₁₋₄ alkyl; and C₃₋₆ cycloalkyl, wherein each C₁₋₄ alkyl, C₃₋₆ cycloalkyl is optionally substituted with one or more halogen;
Optionally at least one of R⁴, R⁵ is a protective group resistant to the reaction conditions of step (a₂);
n is 1 or 2;
m is 0, 1 or 2; and
(a₃)optionally removing the protective group; to give a compound of formula (VI) 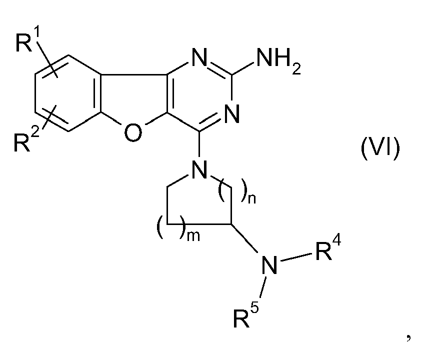 wherein
R¹, R² are independently selected from the group consisting of H; F; Cl; Br; C₁₋₄ alkyl; and OC₁₋₄ alkyl, wherein each C₁₋₄ alkyl is optionally substituted with one or more halogen; and
R⁴, R⁵ are independently selected from the group consisting of H; C₁₋₄ alkyl; and C₃₋₆ cycloalkyl, wherein each C₁₋₄ alkyl, C₃₋₆ cycloalkyl is optionally substituted with one or more halogen;
n is 1 or 2; and
m is 0, 1 or 2.

11. The process according to claim 10, wherein the protective group is the tert.-butyloxycarbonyl group.
